# EUROPEAN PATENT SPECIFICATION

(11) **EP 3 927 399 B1**
(45) Date of publication and mention of the grant of the patent: **19.08.2026**
(21) Application number: 20707037.6
(22) Date of filing: 20.02.2020
(51) Int. Cl.: A61M 5/315, A61M 5/20

(54) **INSULIN DELIVERY ARRANGEMENT AND METHOD FOR INSULIN DOSING**
INSULINVERABREICHUNGSANORDNUNG UND VERFAHREN ZUR INSULINDOSIERUNG
AGENCEMENT D'ADMINISTRATION D'INSULINE ET PROCÉDÉ DE DOSAGE D'INSULINE

(30) Priority: 22.02.2019 EP 19158950
(43) Date of publication of application: 29.12.2021
(73) Proprietor: mySugr GmbH, 1010 Wien (AT)
(72) Inventor: WREDE, Jan, 1010 Wien (AT)
(74) Representative: Wandrey, Anne Julia
(86) International application number: PCT/EP2020/054431
(87) International publication number: WO 2020/169706

(56) References cited:
- WO-A1-2016/019192
- US-A1- 2012 004 637
- US-A1- 2016 117 481
- US-A1- 2018 089 395

## Description

The invention relates to an insulin delivery arrangement comprising a hand-operable insulin delivery device which includes an adjusting unit for presetting of an insulin dose in increments and an applicator unit that can be triggered to deliver or apply the preset insulin dose to a user. The invention further concerns a method for dosing adjustment of such an insulin delivery arrangement.

To date, patients with diabetes employing an insulin injection pen widely use a bolus calculator application on a smart phone or blood glucose meter to get a meal bolus recommendation. The patient therefore has to enter the carbohydrate amounts he wants to eat into the bolus calculator, the bolus calculator then calculates insulin units to be administered and then the patient has to rotate the dial on the pen for the corresponding insulin amount to be injected.

US 2018/0121630 A1 discloses that administration of insulin via insulin (injection) pens has been widely accepted by patients and providers in the ambulatory setting, and in recent years, the inpatient acute care setting as well. Insulin pens offer several advantages over the traditional insulin vial and syringe. A greater degree of comfort at the injection site and ease of use for the patient provide a better experience. Further, a dial on the insulin pen enables dose selection and makes dose accuracy more precise.

US 2016/117481 A1 discloses a system for managing insulin administration or insulin dosing. A method of administering insulin includes receiving subcutaneous information for a patient at a computing device and executing a subcutaneous outpatient program for determining recommended insulin dosages.

WO 2016/019192 A1 discloses an insulin injector with intelligence and communication capabilities that is capable of providing optimized bolus doses of insulin based on information received from a glucose sensor. Embodiments also relate to injectors that communicate data within a health system to provide information to interested parties including the patient and their healthcare provider.

US 2016/0279336 A1 discloses that the patient enables, turns on, or otherwise manipulates an insulin pen to deliver a bolus of fluid to the patient's body as desired throughout the day. For example, at meal time, the patient may utilize the blood glucose meter to obtain a current glucose measurement value, and then turn on the injection pen to initiate a meal bolus. In such embodiments, the patient provides an amount of carbohydrates associated with the meal. For example, the patient may manipulate user interface elements associated with the injection pen to input the carbohydrate amount directly to the injection pen, or alternatively, that patient may manipulate the electronic device to receive and then transfer, to the injection pen, a carbohydrate amount that is input to the client application on the electronic device Additionally, in embodiments where the injection pen does not communicate with the blood glucose meter, the patient may input the current glucose measurement value to the injection pen. Based at least in part on the input carbohydrate amount, the current glucose measurement value, and the stored patient specific parameter values, the injection pen automatically calculates or otherwise determines a meal bolus amount configured to compensate for the carbohydrates in a patient specific manner. When the bolus amount determined by the injection pen is confirmed or otherwise accepted by a user, the injection pen automatically configures its actuation arrangement to precisely deliver the bolus amount automatically in response to manual actuation of a user interface element of the injection pen (e.g., a button associated with fluid delivery).

On this basis an object of the invention is to further improve the known insulin delivery devices and systems and dosing methods and to ease the burden of insulin dosing decisions and number of steps to be taken by the user for injecting a bolus.

The combination of features stated in the independent claims is proposed to achieve this object. Advantageous embodiments and further developments of the invention are derived from the dependent claims.

The invention is based on the idea of determining and displaying to the user, while dialing the insulin-dosage, in parallel an equivalent amount of carbohydrates that can be eaten when the user would inject the dialed units of insulin. Thus, an insulin delivery arrangement or system is proposed in which a dose converter is adapted to receive the presetting or actual setting value for the insulin dose as an input and to provide as an output a carbohydrate value equivalent to an amount of meal carbohydrates which can be compensated by the actual setting for the insulin dose, and wherein a display is provided for displaying the carbohydrate value to the user. In this way, the user can be saved the step of inputting the amount of carbohydrates, but only increment the insulin dose while simultaneously or in parallel an indication of the respective amounts of carbohydrates that would be compensated by the selected dose of insulin is displayed. The user is released from complex mental calculations and/or associated input operations, while at the same time, the insulin delivery device can be manipulated in a simple manner as intended, thus improving glycemic control and treatment satisfaction for patients with diabetes. Thus, the present invention
- can make use of a rotatable dial on existing pens,
- allow faster interaction/input as compared to clicking on a display through a digital menu of a user interface
- can be used in a more flexible manner as compared to e.g. limited buttons for inputting meal size.

In one aspect of the invention an insulin delivery arrangement is disclosed comprising
- a hand-operable insulin delivery device being an insulin injection pen which includes an adjusting unit for presetting of an insulin dose and an applicator unit that can be triggered to deliver the preset insulin dose to a user, wherein the adjusting unit includes a stepwise rotatable dial
   characterized by
- a dose converter being part of the insulin injection pen adapted to receive the presetting for the insulin dose as an input and to provide as an output a carbohydrate value equivalent to an amount of meal carbohydrates which can be compensated by the respective insulin dose, wherein the dose converter is formed by a digital processor or by an analog mechanism and has a routine to determine the carbohydrate value based on the presetting value for the insulin dose and at least an insulin to carbohydrate ratio, and
- a display arranged on the surface of the insulin injection pen for displaying the carbohydrate value to the user, wherein the carbohydrate value is changed on the display synchronously to a rotation of the dial.

The hand-operable insulin delivery device in the present invention can thus do without an electrically operated dose setting member (which, e.g., would receive input via touch screen or plus/minus buttons). Preferably, a rotatable dial is provided for presetting the insulin dose in a plurality of steps or increments, more preferred in at least 5 steps.

A further improvement provides that the display is provided as a digital screen to display the carbohydrate values in parallel to the dialed insulin units. For this purpose, it may also be conceivable to employ an analog sliding scale.

A still further improvement in this direction provides that the adjusting unit is formed as a stepwise rotatable dial, and that the carbohydrate value is simultaneously changed on the display corresponding to a degree of rotation of the dial.

In this connection, it is also advantageous if the display is arranged specifically on an end face of the generally cylindrical insulin injection pen.

According to an example not forming part of the invention, a remote mobile device, preferably a smart phone or a smart watch, which is arranged separate from the insulin delivery device, wherein the mobile device is adapted to wirelessly communicate with the insulin delivery device and preferably includes the dose converter as a mobile application. In this way, widely-used smart wearable devices can be used to fulfil additional resource demanding tasks.

In particular, when rotating the dial for the insulin-dosage on an insulin pen, a signal is transmitted to the remote device that starts the remote bolus or dose converter. If the pen transmits insulin dosage information, the converter unit calculates the equivalent amount of carbohydrates and displays it.

In this connection, it is advantageous when the display for displaying the carbohydrate value is provided on the mobile device. It is also conceivable to additionally or alternatively use the display of another connected wearable device like a smart watch or fitness tracker.

A further improvement in user handling provides that the remote device is configured to automatically retrieve and process the actual setting for the insulin dose upon a user manipulation of the insulin delivery device. In one embodiment, the remote device is thus configured to automatically retrieve and process the actual setting for the insulin dose upon a rotation of the dial. In another embodiment, the insulin delivery device is configured to automatically send the actual setting for the insulin dose to the remote mobile device upon a rotation of the dial.

As a still further advantageous measure, a glucose meter, in particular a continuous monitoring glucose meter, is configured to provide a glucose measurement value to the dose converter. Thereby, it is possible to additionally consider glucose information for improved bolus calculation.

One or more advantageous embodiments may further comprise that the glucose measurement value is retrieved from a glucose meter by means of the remote device, and that the glucose measurement value and, where appropriate, other patient specific data are transmitted from the remote device to the insulin delivery device.

Another improvement in this direction provides that the dose converter is configured to include a user-specific correction bolus in a calculation of the carbohydrate value.

For improved use convenience, it is advantageous if the insulin delivery device has an activation circuit which triggers transmission of user specific parameters from the remote device when the insulin delivery device is activated.

In particular, while dialling the insulin-dosage, an insulin pen in parallel displays the equivalent amount of carbohydrates that can be compensated in a meal. The equivalent carbohydrate amount is calculated using a pen-integrated dose converter that also considers cases of elevated glucose which require a correction bolus, where glucose data and patient specific parameters for bolus calculation are transmitted from a remote device to the pen.

In even more sophisticated embodiments, the dose converter has a software routine to determine the carbohydrate value based on the actual setting value for the insulin dose and at least one of a measured glucose value, a target glucose value, an insulin to carbohydrate ratio, an insulin sensitivity factor and a basal insulin dose.

Another aspect of the invention concerns a method for dosing adjustment of an insulin delivery arrangement comprising the steps of
- presetting of an insulin dose in an insulin injection pen by user manipulation of an adjusting unit comprising a stepwise rotatable dial,
- automatically providing the presetting for the insulin dose as an input to a dose converter without additional user interaction,
- receiving as an output from the dose converter a carbohydrate value equivalent to an amount of meal carbohydrates which can be compensated by the respective insulin dose, wherein the carbohydrate value is determined by the routine of the dose converter, and
- displaying on a display the carbohydrate value in parallel tc the presetting for the insulin dose, wherein the carbohydrate value is changed on the display synchronously to a rotation of the dial.

In this way, the same advantages are achieved as detailed above in connection to the insulin delivery systems.

In the following, the invention is further elucidated on the basis of embodiment examples shown schematically in the drawings, where
- Fig. 1: depicts in a perspective view an arrangement of an insulin injection pen and a dose converter for calculating an amount of carbohydrate equivalent to an adjusted insulin dose;
- Fig. 2: shows a proximal end of the insulin pen including a digital display to indicate the carbohydrate value; and
- Fig. 3: illustrates a further exemplary insulin delivery system comprising an insulin pen, a smartphone and a glucose meter.

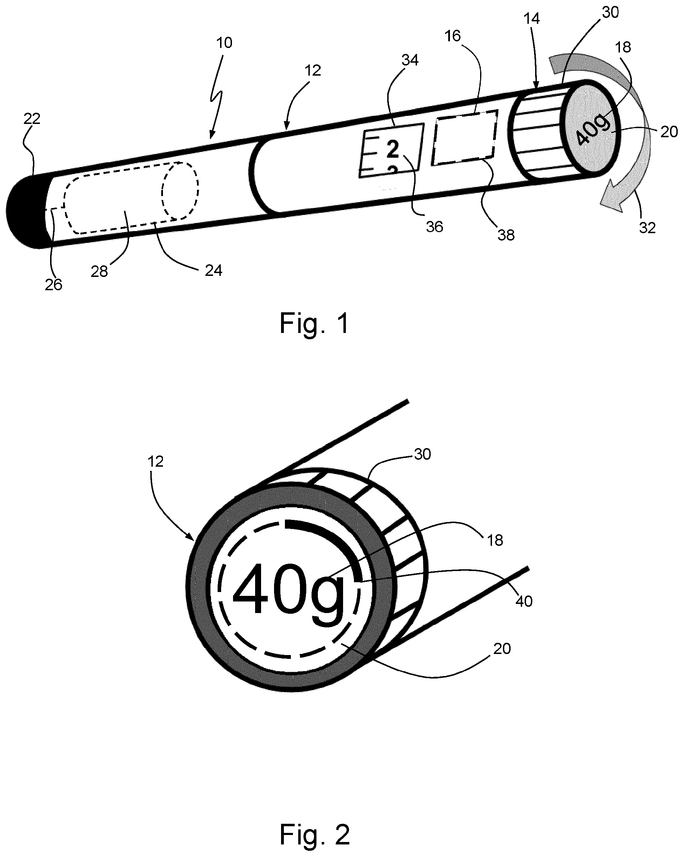

As depicted in Fig. 1, an exemplary embodiment of an insulin delivery arrangement 10 comprises an insulin pen 12 having an adjusting unit 14 for presetting of an insulin dose and further comprising a dose converter 16 schematically illustrated as being integrated in the insulin pen 12, wherein the dose converter 16 is adapted to receive an actual setting value for the insulin dose as an input and to provide an equivalent carbohydrate value as an output 18 on a digital display 20.

The insulin pen 12 includes a removable pen cap 22 to protect an applicator unit 24 illustrated schematically in broken lines as comprising a distal projecting injection needle 26 and an insulin cartridge 28.

The adjusting unit 14 comprises a manually rotatable dial 30 for a user to preset an insulin dosage to be delivered upon final triggering of the applicator unit 24. The dial 30 is formed as a dose knob which can be turned forward or backward (arrow 32) to increase or decrease the insulin dose. For feedback to the user on the actual setting, the adjusting unit 14 further comprises a dose counter 34 as an analog sliding scale which is connected to the dial 30, where the adjusted insulin dose 36 lines up in the center.

In exemplary embodiments described herein, the dose converter 16 includes a digital processor 38 which receives an actual setting or rotational position of the dial 30 as an input value. As explained in more detail below, the dose converter 16 then calculates a carbohydrate value equivalent to an amount of carbohydrates in a meal which can be compensated by the insulin dose according to the actual setting.

As depicted in Fig. 2, the carbohydrate value or output 18 of the dose converter 16 is indicated in digital form on the display screen 20 on the proximal end of the pen 12. Thus, the user can directly take notice of the carbohydrate value while turning the dial 30 to find the appropriate setting. The display 20 may also provide additional information, such as a dose memory 40 to visualize insulin units last injected. It is also conceivable that the display 20 is arranged on the long side of the pen 12 preferably in the vicinity of the scale 34.

In alternative embodiments, the dose converter 16 may be simply provided by an analog mechanism which is mechanically coupled to the dial 14 on the input side and further coupled to an analog graduated scale to show the carbohydrate value similar to the dose counter 34.

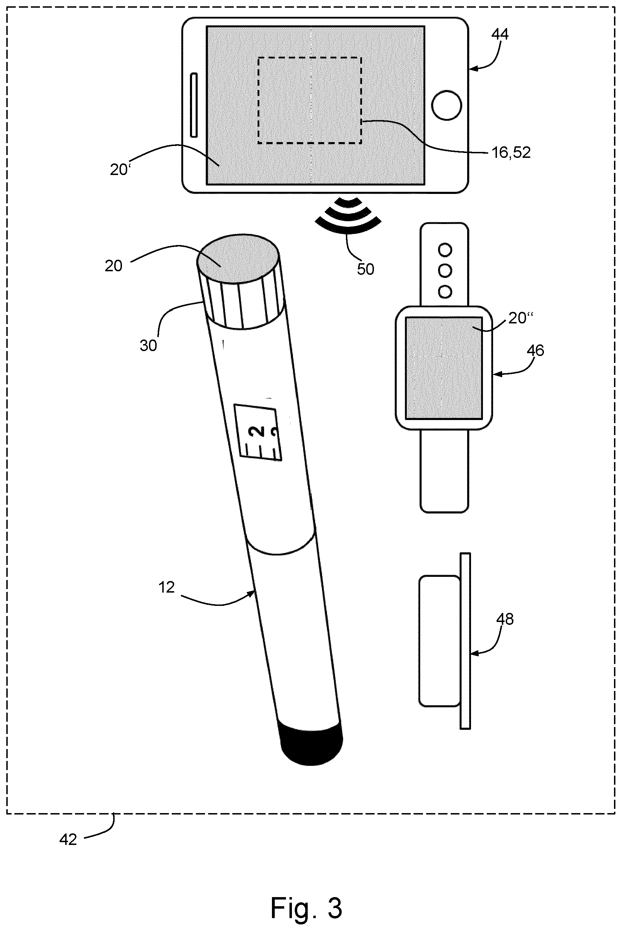

Fig. 3 depicts a more enhanced insulin delivery system 42 comprising an insulin pen 12, a primary remote mobile device 44, if needed a secondary mobile device 46, and a glucose meter 48.

The primary mobile device 44 is shown as a smartphone, whereas the secondary device may be a smart watch or a fitness tracker. The shown devices 12,46,48 are in wireless communication with the smart phone 44 e.g. using Bluetooth protocols, as illustrated by radio signals 50 in Fig. 3.

In some cases, the smartphone 44 can include the dose converter 16 as a mobile application 52 running on the internal processor. In exemplary embodiments, the screen of the smartphone may be used as the display 20' for indicating the calculated carbohydrate value 18 to the user. For further user convenience, the smartphone 44 may automatically retrieve and process the actual setting for the insulin dose upon a user manipulation of the insulin pen 12. The synchronization may occur with indexed positions or clicks from the dial 30 to provide haptic or acoustic feedback to the user.

The glucose meter 48 is illustrated as a body wearable instrument which allows continuous measurements of glucose independent of user interaction. In other cases, the mobile device 44 may prompt the user to take a measurement and to transmit or input the glucose measurement value.

In further embodiments of the insulin delivery system, when the insulin pen 12 is activated, glucose data and patient specific parameters for bolus calculation are transmitted from the smartphone 44 to the pen 12. Glucose data is transmitted to the smartphone from the connected glucose meter 48. Such a communication may be triggered by an activation circuit (not shown) on the pen 12. Then, the display 20 of the pen 12 can be used to indicate glucose data, the insulin dosage and the equivalent carbohydrate value.

Upon calculation of the carbohydrate value, the dose converter 16 may include a user specific correction bolus, based on a difference between a current and a target (blood) glucose value. For example, if the current blood glucose is 200 mg/dl and the target blood glucose is 150 mg/dl, then the output of the carbohydrate value will remain at Zero for the first steps of rotation of the dial 30, as the corresponding amount of insulin is needed only for lowering blood glucose.

The simplest embodiment might use only one (user-defined) insulin-to-carbohydrate ratio to indicate for the patient how many grams of carbohydrates one unit of insulin can compensate for. This may be implemented in a mechanical bolus converter by a simple sliding scale.

In a more advanced embodiment, different insulin-to-carbohydrate ratios might be defined depending on the time of the day (e.g., morning, lunch, dinner time) for the patient. If the current glucose value is considered in the bolus calculation, a patient-specific insulin sensitivity factor might be considered which reflects the patient's sensitivity to insulin (e.g., an amount of drop in glucose level per unit of insulin administered). If the glucose value is below target level at lunch time when user wants to eat certain amount of carbohydrate, he would have to inject less insulin to bring his glucose back in target level than in a situation where the current glucose value was normal or even above the target level.

For the latter option, it is preferable to display the information on the smart watch 46 in addition or alternatively to the smartphone **44.** In this case the user would only have to take out the pen 12 from his pocket (not the smartphone) and have a look on the display **20"** of his smart watch 46 showing the amount of carbohydrates he may eat with the dialled amount of insulin.

In a more advanced calculation routine, the dose converter 16 has a software routine to determine the carbohydrate value (Ct) based on the actual setting value for the insulin dose (It) and other user events and/or parameters, such as a measured glucose value Gₜ, a target glucose value T_{g}, an insulin to carbohydrate ratio ICR **(i.e.** amount of carbohydrates needed to match the glucose lowering effect of one unit U of insulin, given in U/g), and an insulin sensitivity factor ISF (as a measure for the decrease in glucose level caused by one unit of administered insulin, given in U/(mg/dl).

The carbohydrate value then may be calculated as follows: ${C}_{t} = \left({I}_{t}-\left({G}_{t}-{T}_{g}\right)*ISF\right) / ICR$

In one embodiment, dose converter 16 has a more general software routine. The carbohydrate value (Ct) may be determined as follows: ${C}_{t} = f \left({I}_{t}, {G}_{t}, {T}_{t}, {Φ}_{t}, {θ}_{t}\right)$
wherein It is the setting value for the insulin dose at the actual time t, Gₜ is the actual measured glucose value, Tₜ is the actual target glucose value,
and wherein ${Φ}_{t} = \left[\left({C}_{t 1}, {I}_{t 1}, {G}_{t 1},…\right), …, \left({C}_{tn}, {I}_{tn}, {G}_{tn},…\right)\right]$
describes the history of user events (e.g., meals (indicated as ingested carbohydrates C), insulin injections I, measured glucose values G) from a past timepoint t1 until a cutoff time tn in the past,
and wherein ${θ}_{t} = \left[\left({ISF}_{t}, {ICR}_{t}, {T}_{t}, …\right), …, \left({ISF}_{tn}, {ICR}_{tn}, {T}_{tn}, …\right)\right]$
wherein θₜ describes the history of user parameters (e.g, insulin sensitivity factor ISF, insulin to carbohydrate ratio ICR, target glucose value T) from timepoint t1 until cutoff time tn in the past.

A variation of parameters over time can thus be considered. E.g., different insulin-to-carbohydrate ratios might be defined depending on the time of the day for the patient.

If needed, further events and parameters (indicated as "..." in the equations (3) and (4) above) than those exemplified above, such as a basal insulin dose, body weight and physiologic activity can also be considered.

In general, it is known in the art to match meal insulin to carbohydrate intake for insulin bolus size calculation, which is carbohydrate driven in the way that the user has to do bolus calculations. In contrast, the insulin driven approach of the invention allows much simpler interaction.

## Claims

1. An insulin delivery arrangement (10;42) comprising
- an insulin injection pen (12) which includes an adjusting unit (14) for manual presetting of an insulin dose and an applicator unit (24) that can be triggered to deliver the preset insulin dose to a user, wherein the adjusting unit (14) includes a stepwise rotatable dial (30)
**characterized by**
- a dose converter (16) being part of the insulin injection pen adapted to receive the presetting for the insulin dose as an input and to provide as an output a carbohydrate value equivalent to an amount of meal carbohydrates which can be compensated by the respective insulin dose, wherein the dose converter (16) is formed by a digital processor or by an analog mechanism and has a routine to determine the carbohydrate value based on the presetting value for the insulin dose and at least an insulin to carbohydrate ratio, and
- a display (20,20',20") arranged on the surface of the insulin injection pen for displaying the carbohydrate value to the user
- wherein the carbohydrate value is changed on the display (20,20',20") synchronously to a rotation of the dial (30).

2. The arrangement of claim 1, wherein the display (20,20',20") is provided as a digital screen or as an analog sliding scale.

3. The arrangement according to any of claim 1 or 2, further comprising a remote mobile device (44,46), preferably a smart phone and/or a smart watch, which is arranged separate from the insulin injection pen (12), wherein the mobile device (44) is adapted to wirelessly communicate with the insulin injection pen (12).

4. The arrangement according to any of claims 1 to 3, further comprising a glucose meter (48), in particular a continuous monitoring glucose meter, which is configured to provide a glucose measurement value to the dose converter (16).

5. The arrangement of claim 4, wherein the glucose measurement value is retrieved from the glucose meter (48) by means of the remote mobile device (44,46), and wherein the glucose measurement value and, where appropriate, other patient specific data are transmitted from the remote mobile device to the insulin injection pen (12).

6. The arrangement according to any of claims 4 or 5, wherein the dose converter (16) is configured to include a user-specific correction bolus in a calculation of the carbohydrate value.

7. The arrangement according to claim 3, wherein the insulin delivery device (12) has an activation circuit which triggers transmission of user specific parameters from the remote mobile device (44,46) when the insulin injection pen (12) is activated.

8. The arrangement according to any of claims 1 to 7, wherein the routine to determine the carbohydrate value is based additionally on at least one of a measured glucose value, a target glucose value, an insulin sensivity factor and a basal insulin dose.

9. Method for dosing adjustment of an insulin delivery arrangement (10) according to claim 1 comprising the steps of
- presetting of an insulin dose in the insulin injection pen (12) by user manipulation of the adjusting unit (14) comprising the stepwise rotatable dial (30),
**characterized by**
- automatically providing the presetting for the insulin dose as an input to the dose converter (16) without additional user interaction,
- receiving as an output from the dose converter (16) a carbohydrate value equivalent to an amount of meal carbohydrates which can be compensated by the respective insulin dose, wherein the carbohydrate value is determined by the routine of the dose converter (16), and
- displaying on the display (20,20',20") the carbohydrate value in parallel to the presetting for the insulin dose, wherein the carbohydrate value is changed on the display (20,20',20") synchronously to a rotation of the dial (30).

## Patentansprüche

1. Insulinverabreichungsanordnung (10; 42), umfassend
- einen Insulininjektionsstift (12), der eine Einstelleinheit (14) zur manuellen Voreinstellung einer Insulindosis und eine Applikatoreinheit (24) einschließt, die ausgelöst werden kann, um die voreingestellte Insulindosis an einen Benutzer zu verabreichen, wobei die Einstelleinheit (14) eine schrittweise drehbare Einstellscheibe (30) einschließt,
**gekennzeichnet durch**
- einen Dosisumwandler (16), der Teil des Insulininjektionsstifts ist und dazu ausgelegt ist, die Voreinstellung für die Insulindosis als Eingabe zu empfangen und als Ausgabe einen Kohlenhydratwert bereitzustellen, der einer Menge an Mahlzeitkohlenhydraten entspricht, die durch die jeweilige Insulindosis kompensiert werden kann, wobei der Dosisumwandler (16) durch einen digitalen Prozessor oder durch einen analogen Mechanismus gebildet wird und eine Routine aufweist, um den Kohlenhydratwert basierend auf dem Voreinstellungswert für die Insulindosis und mindestens einem Insulin-Kohlenhydrat-Verhältnis zu bestimmen, und
- eine Anzeige (20, 20', 20"), die auf der Oberfläche des Insulininjektionsstifts angeordnet ist, um dem Benutzer den Kohlenhydratwert anzuzeigen,
- wobei der Kohlenhydratwert auf der Anzeige (20, 20', 20") synchron zu einer Drehung der Einstellscheibe (30) geändert wird.

2. Anordnung nach Anspruch 1, wobei die Anzeige (20, 20', 20") als digitaler Bildschirm oder als analoge Gleitskala bereitgestellt ist.

3. Anordnung nach einem der Ansprüche 1 oder 2, ferner umfassend ein entferntes Mobilgerät (44, 46), vorzugsweise ein Smartphone und/oder eine Smartwatch, das getrennt von dem Insulininjektionsstift (12) angeordnet ist, wobei das Mobilgerät (44) dazu ausgelegt ist, drahtlos mit dem Insulininjektionsstift (12) zu kommunizieren.

4. Anordnung nach einem der Ansprüche 1 bis 3, ferner umfassend ein Glukosemessgerät (48), insbesondere ein Messgerät zur kontinuierlichen Glukoseüberwachung, das dazu konfiguriert ist, dem Dosisumwandler (16) einen Glukosemesswert bereitzustellen.

5. Anordnung nach Anspruch 4, wobei der Glukosemesswert von dem Glukosemessgerät (48) mittels des entfernten mobilen Geräts (44, 46) abgerufen wird und wobei der Glukosemesswert und gegebenenfalls andere patientenspezifische Daten von dem entfernten mobilen Gerät an den Insulininjektionsstift (12) übertragen werden.

6. Anordnung nach einem der Ansprüche 4 oder 5, wobei der Dosisumwandler (16) dazu konfiguriert ist, einen benutzerspezifischen Korrekturbolus in eine Berechnung des Kohlenhydratwerts einzubeziehen.

7. Anordnung nach Anspruch 3, wobei die Insulinverabreichungsvorrichtung (12) eine Aktivierungsschaltung aufweist, die eine Übertragung benutzerspezifischer Parameter von dem entfernten Mobilgerät (44, 46) auslöst, wenn der Insulininjektionsstift (12) aktiviert ist.

8. Anordnung nach einem der Ansprüche 1 bis 7, wobei die Routine zum Bestimmen des Kohlenhydratwerts zusätzlich auf mindestens einem aus einem gemessenen Glukosewert, einem Zielglukosewert, einem Insulinempfindlichkeitsfaktor und einer Basalinsulindosis basiert.

9. Verfahren zur Dosierungseinstellung einer Insulinverabreichungsanordnung (10) nach Anspruch 1, umfassend die folgenden Schritte:
- Voreinstellen einer Insulindosis in dem Insulininjektionsstift (12) durch Betätigung der Einstelleinheit (14), die die schrittweise drehbare Einstellscheibe (30) umfasst, durch den Benutzer,
**gekennzeichnet durch**
- automatisches Bereitstellen der Voreinstellung für die Insulindosis als eine Eingabe in den Dosisumwandler (16) ohne zusätzliche Benutzerinteraktion,
- Empfangen eines Kohlenhydratwerts, der einer Menge an Mahlzeit-Kohlenhydraten entspricht, die durch die jeweilige Insulindosis kompensiert werden kann, als Ausgabe von dem Dosisumwandler (16), wobei der Kohlenhydratwert durch die Routine des Dosisumwandlers (16) bestimmt wird, und
- Anzeigen des Kohlenhydratwerts auf der Anzeige (20, 20', 20") parallel zu der Voreinstellung für die Insulindosis, wobei der Kohlenhydratwert auf der Anzeige (20, 20', 20") synchron zu einer Drehung der Einstellscheibe (30) geändert wird.

## Revendications

1. Agencement d'administration d'insuline (10 ; 42) comprenant
- un stylo d'injection d'insuline (12) qui comprend une unité de réglage (14) pour le préréglage manuel d'une dose d'insuline et une unité d'applicateur (24) qui peut être déclenchée pour administrer la dose d'insuline préréglée à un utilisateur, dans lequel l'unité de réglage (14) comprend une molette (30) pouvant tourner pas à pas
**caractérisé par**
- un convertisseur de dose (16) faisant partie du stylo d'injection d'insuline adapté pour recevoir le préréglage pour la dose d'insuline en tant qu'entrée et pour fournir en tant que sortie une valeur de glucides équivalente à une quantité de glucides de repas qui peut être compensée par la dose d'insuline respective, le convertisseur de dose (16) étant formé par un processeur numérique ou par un mécanisme analogique et a une routine pour déterminer la valeur de glucides en se basant sur la valeur de préréglage pour la dose d'insuline et au moins un rapport insuline à glucides, et
- un affichage (20, 20', 20") agencé sur la surface du stylo d'injection d'insuline pour afficher la valeur de glucides à l'utilisateur
- dans lequel la valeur de glucides est modifiée sur l'affichage (20, 20', 20") de manière synchrone avec une rotation de la molette (30).

2. Agencement selon la revendication 1, dans lequel l'affichage (20, 20', 20") est fourni en tant qu'écran numérique ou en tant qu'échelle coulissante analogique.

3. Agencement selon l'une quelconque des revendications 1 ou 2, comprenant en outre un dispositif mobile distant (44, 46), de préférence un smartphone et/ou une montre connectée, qui est agencé séparément du stylo d'injection d'insuline (12), dans lequel le dispositif mobile (44) est adapté pour communiquer sans fil avec le stylo d'injection d'insuline (12).

4. Agencement selon l'une quelconque des revendications 1 à 3, comprenant en outre un glucomètre (48), en particulier un glucomètre pour surveillance en continu, qui est configuré pour fournir une valeur de mesure de glucose au convertisseur de dose (16).

5. Agencement selon la revendication 4, dans lequel la valeur de mesure de glucose est récupérée du glucomètre (48) au moyen du dispositif mobile distant (44, 46), et dans lequel la valeur de mesure de glucose et, lorsque cela est approprié, d'autres données spécifiques au patient sont transmises du dispositif mobile distant au stylo d'injection d'insuline (12).

6. Agencement selon l'une quelconque des revendications 4 ou 5, dans lequel le convertisseur de dose (16) est configuré pour comprendre un bolus de correction spécifique à l'utilisateur dans un calcul de la valeur de glucides.

7. Agencement selon la revendication 3, dans lequel le dispositif d'administration d'insuline (12) a un circuit d'activation qui déclenche la transmission de paramètres spécifiques à l'utilisateur depuis le dispositif mobile distant (44, 46) lorsque le stylo d'injection d'insuline (12) est activé.

8. Agencement selon l'une quelconque des revendications 1 à 7, dans lequel la routine pour déterminer la valeur de glucides se base de plus sur au moins une parmi une valeur de glucose mesurée, une valeur de glucose cible, un facteur de sensibilité à l'insuline et une dose d'insuline basale.

9. Procédé de réglage de dosage d'un agencement d'administration d'insuline (10) selon la revendication 1 comprenant les étapes de
- préréglage d'une dose d'insuline dans le stylo d'injection d'insuline (12) par manipulation par l'utilisateur de l'unité de réglage (14) comprenant la molette (30) pouvant tourner pas à pas,
**caractérisé par**
- la fourniture de manière automatique du préréglage pour la dose d'insuline en tant qu'entrée au convertisseur de dose (16) sans interaction supplémentaire de l'utilisateur,
- la réception en tant que sortie du convertisseur de dose (16) d'une valeur de glucides équivalente à une quantité de glucides de repas qui peut être compensée par la dose d'insuline respective, la valeur de glucides étant déterminée par la routine du convertisseur de dose (16), et
- l'affichage sur l'affichage (20, 20', 20") de la valeur de glucides en parallèle au préréglage de la dose d'insuline, la valeur de glucides étant modifiée sur l'affichage (20, 20', 20") de manière synchrone avec une rotation de la molette (30).
